# EUROPÄISCHE PATENTSCHRIFT

(11) **EP 1 165 704 B1**
(45) Veröffentlichungstag und Bekanntmachung des Hinweises auf die Patenterteilung: **02.06.2004**
(21) Anmeldenummer: 00922544.2
(22) Anmeldetag: 23.03.2000
(51) Int. Cl.: C09D 5/03, C09D 7/12, G03F 7/031, C07C 69/738

(54) **VERWENDUNG VON PHENYLGLYOXALSÄUREESTERN ALS PHOTOINITIATOREN**
UTILIZATION OF PHENYLGLYOXILIC ACID ESTERS AS PHOTOINITIATORS
UTILISATION D'ESTERS D'ACIDE PHENYLGLYOXALIQUE COMME PHOTO-INITIATEURS

(30) Priorität: 24.03.1999 DE 19913353
(43) Veröffentlichungstag der Anmeldung: 02.01.2002
(73) Patentinhaber: BASF AKTIENGESELLSCHAFT, 67056 Ludwigshafen (DE)
(72) Erfinder: SCHWALM, Reinhold, D-67157 Wachenheim (DE); KÖNIGER, Rainer, D-67251 Freinsheim (DE)
(74) Vertreter: Kinzebach, Werner, Dr.
(86) Internationale Anmeldenummer: PCT/EP2000/002609
(87) Internationale Veröffentlichungsnummer: WO 2000/056822

(56) Entgegenhaltungen:
- WO-A-98/33761
- US-A- 4 279 720

## Beschreibung

Die vorliegende Erfindung betrifft die Verwendung von bestimmten Phenylglyoxalsäureestern als Photoinitiatoren in Pulverlacken für Außenanwendungen.

Konventionelle strahlungshärtbare Lacke enthalten in der Regel reaktive Monomere zur Einstellung der Applikationsviskosität (Reaktivverdünner) und werden bei Raumtemperatur gehärtet. Diese Lacke haben den Nachteil, dass die als Reaktivverdünner verwendeten Monomere in poröse Untergründe, wie Holz oder Papier, eindringen können und damit der UV-Härtung entzogen sind. Außerdem können bei der Belichtung von dreidimensionalen Objekten Schattenbereiche auftreten, die nur unzureichend belichtet werden, so dass flüssige Lacke in diesen Bereichen klebrig bleiben können. Man hat daher wässrige UV-Lacke entwickelt, welche zur Trocknung thermisch behandelt und bei höheren Temperaturen gehärtet werden, siehe W. Reich et al., RadTech 98, Conference Proceedings, Chicago 1998, 258-265.

Zur Vermeidung der erwähnten Probleme sind weiter UV-härtbare Pulverlacke vorgeschlagen worden, welche nach dem Auftragen auf das Substrat mittels konventioneller Methoden bei höheren Temperaturen aufgeschmolzen und gehärtet werden, siehe beispielsweise RadTech, Conference Proceedings, Chicago 1998, 170-176; JOT 1998, 2, 44-47; PPCJ 1997, (9), 18,20; EP 636 669 A; 650 985 A; 650 978 A und US 5,639,560. Ferner sind Dual Cure-Systeme bekannt, bei denen die Härtung durch Strahlung und Luftfeuchtigkeit erfolgt (US 4,138,299; 4,173,682; 4,415,604 und EP 549 116 A) oder welche neben einem UV-Radikalinitiator auch einen thermisch aktivierbaren Initiator enthalten (EP 844 286 A).

Es hat sich gezeigt, dass viele der üblichen Photoinitiatoren unter der thermischen Belastung des Aufschmelzens von Pulverlacken zu flüchtig sind, um eine Härtung durch UV-Bestrahlung zu ermöglichen. Weiter ist die Verarmung an Photoinitiatoren an der Lackoberfläche besonders unerwünscht, weil dort die stärkste Sauerstoffinhibierung erfolgt, so dass eine unzureichende Härtung und damit schlechte Filmeigenschaften resultieren, wie niedrige Chemikalienbeständigkeit und unzureichende Blockfestigkeit. Man hat daher die weniger flüchtigen Ester der Arylglyoxalsäure als Photoinitiatoren in Betracht gezogen, siehe beispielsweise DE 26 39 742 A, US 4,024,297 und die ältere, nachveröffentlichte DE 198 26 712 A. Polymergebundene Arylglyoxylate sind aus der DE 28 25 955 A und Macromolecules 1998, 31, 322-327, bekannt.

Die WO 98/33761 beschreibt Diester von Arylglyoxalsäuren mit Diolen. Diese Ester sind Photoinitiatoren mit geringer Flüchtigkeit, die in flüssigen Systemen zur Anwendung kommen. Ihre Brauchbarkeit in Pulverlacken ist lediglich allgemein erwähnt.

Überraschenderweise wurde nun gefunden, dass bestimmte Ester von Arylglyoxalsäuren mit Diolen als Photoinitiatoren in Pulverlacken für Außenanwendungen besonders geeignet sind. Insbesondere hat sich gezeigt, dass die erfindungsgemäß verwendeten Ester vor der Bestrahlung ohne Nachteile thermisch belastet werden können. Außerdem wurde gefunden, dass die Photoinitiatoren besonders vergilbungsarm sind.

Gegenstand der vorliegenden Erfindung ist daher die Verwendung von Phenylglyoxalsäureestern der Formel I 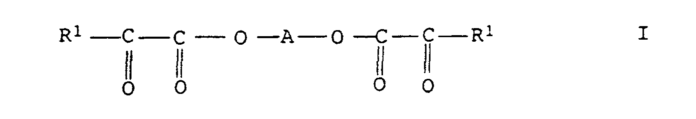 worin die beiden Reste R¹ unabhängig voneinander für einen Rest der Formel 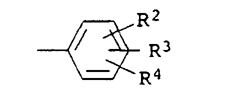 stehen,
R², R³ und R⁴ unabhängig voneinander für H, C₁-C₆-Alkyl, das gegebenenfalls durch OH, OC₁-C₆-Alkyl oder OCOC₁-C₆-Alkyl substituiert ist, oder für OH oder OC₁-C₆-Alkyl stehen;
A für C₂-C₆-Alkylen oder einen Rest der Formeln 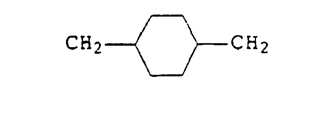 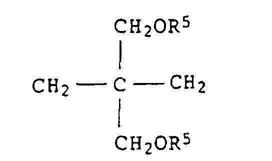 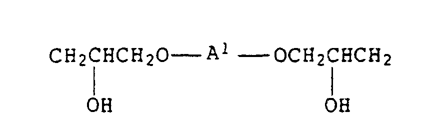 steht,
die Reste R⁵ unabhängig voneinander für H oder COCOR¹ stehen und
A¹ für C₂-C₆-Alkylen oder 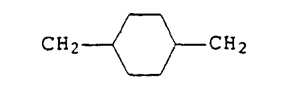 steht,
als Photoinitiatoren in Pulverlacken für Außenanwendungen.

C₁-C₆-Alkyl bedeutet eine geradkettige oder verzweigte Alkylgruppe mit 1 bis 6 Kohlenstoffatomen, vorzugsweise 1 bis 4 Kohlenstoffatomen. Beispiele für Alkylgruppen sind Methyl, Ethyl, Propyl, Isopropyl, n-Butyl, sec-Butyl, iso-Butyl, tert-Butyl, Pentyl und Hexyl.

C₂-C₆-Alkylen steht für eine geradkettige oder verzweigte Alkylengruppe mit 2 bis 6 Kohlenstoffatomen. Beispiele für Alkylengruppen sind Methylen, Ethylen, Propylen, Isopropylen, n-Butylen, sec-Butylen, Isobutylen, tert-Butylen, Pentylen und Hexylen.

Vorzugsweise steht einer der Reste R², R³ und R⁴ für C₁-C₆-Alkyl und die beiden anderen für H, und besonders bevorzugt stehen R², R³ und R⁴ alle für H.

A steht vorzugsweise für 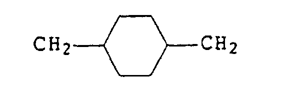 oder 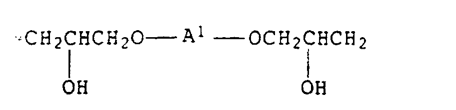 wobei A¹ für 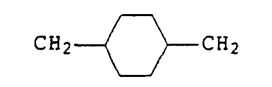 steht. Die Verbindungen der Formel I, worin A die genannten bevorzugten Bedeutungen hat, sind ebenfalls Gegenstand der Erfindung.

Die Verbindungen der Formel I sind entweder bekannt oder können nach den in der WO 98/33761 beschriebenen Methoden hergestellt werden. Außerdem ist es möglich, die gewünschte Arylglyoxalsäure mit dem Alkohol in Anwesenheit eines sauren Katalysators, wie Schwefelsäure, p-Toluolsulfonsäure etc., nach Art einer klassischen Veresterung zu dem gewünschten Ester umzusetzen. Die Veresterung wird zweckmäßigerweise in einem mit Wasser nicht mischbaren Lösungsmittel, beispielsweise ein Kohlenwasserstoff, wie Methylcyclohexan, Toluol, Xylol etc., durchgeführt. Das gebildete Wasser wird in üblicher weise aus dem Reaktionsgemisch entfernt.

Die Verbindungen der Formel I, worin A für 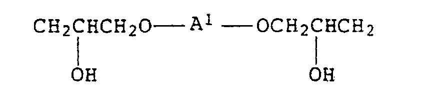 steht, können hergestellt werden durch Umsetzung der entsprechenden Arylglyoxalsäure mit einem Diglycidylether der Formel 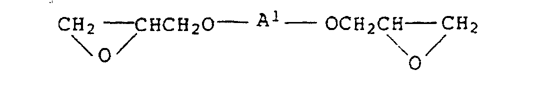 worin A¹ die oben angegebenen Bedeutungen besitzt. Die Umsetzung wird im Allgemeinen ohne Lösungsmittel und in Gegenwart eines geeigneten Katalysators, wie ein Tetraalkylammoniumhalogenid, durchgeführt. Die Reaktionstemperatur liegt im Allgemeinen im Bereich von 80 bis 120 °C.

Die Verbindungen der Formel I sind als Photoinitiatoren für die Photopolymerisation ethylenisch ungesättigter Verbindungen brauchbar. Insbesondere sind sie als Photoinitiatoren in Pulverlacken für Innen- und vorzugsweise Außenanwendungen geeignet. Sie können alleine oder zusammen mit anderen Photoinitiatoren zur Anwendung kommen. Photoinitiatoren, mit welchen sie im Gemisch eingesetzt werden können, sind beispielsweise Mono- oder Bisacylphosphinoxide, Benzophenone oder Hydroxyacetophenone. Weitere andere Photoinitiatoren sind beispielsweise in der WO 98/33761 beschrieben.

Die Verbindungen der Formel I kommen im Allgemeinen in strahlungshärtbaren Zusammensetzungen zur Anwendung. Diese umfassen wenigstens eine ethylenisch ungesättigte, strahlungshärtbare Substanz und wenigstens eine Verbindung der Formel I als Photoinitiator sowie gegebenenfalls weitere Photoinitiatoren und/oder Additive. Die Menge an Verbindungen der Formel I in den Zusammensetzungen liegt im Allgemeinen im Bereich von 0,1 bis 10 Gew.-%, vorzugsweise 0,5 bis 8 Gew.-%, bezogen auf das Gewicht der strahlungshärtbaren Substanz. Soweit Gemische mit anderen Photoinitiatoren zur Anwendung kommen, enthalten die Gemische im Allgemeinen 0,1 bis 50 Gew.-%, bezogen auf das Gesamtgewicht des Gemisches, an den anderen Photoinitiatoren.

Die Menge an strahlungshärtbarer Substanz in den Zusammensetzungen liegt im Allgemeinen im Bereich von 10 bis 90 Gew.-%, vorzugsweise 30 bis 90 Gew.-%, bezogen auf das Gesamtgewicht der Zusammensetzung. Im Falle von Pulverlacken liegt die Menge an strahlungshärtbaren Substanzen im Allgemeinen im Bereich von 50 bis 90 Gew.-%, vorzugsweise 60 bis 90 Gew.-%, bezogen auf das Gesamtgewicht der Zusammensetzung.

Die strahlungshärtbare Substanz kann eine oder mehrere olefinische, radikalisch polymerisierbare Doppelbindungen aufweisen und monomerer oder polymerer, insbesondere oligomerer Natur sein. Beispiele für monomere Substanzen (Reaktivverdünner) mit einer Doppelbindung sind Alkyl- oder Hydroxyalkylacrylate oder -methacrylate, wie Methyl-, Ethyl-, n-Butyl-, 2-Ethylhexylacrylat oder - methacrylat, und 2-Hydroxyethylacrylat oder -methacrylat. weitere Beispiele sind (Meth)acrylnitril, (Meth)acrylamid, N-C₁-C₄-alkylsubstituierte (Meth)acrylamide, Vinylester, wie Vinylacetat, Vinylether, wie Isobutylvinylether, Vinylaromaten, wie Styrol, N-Vinylpyrrolidon, Vinylchlorid und Vinylidenchlorid. Beispiele für Monomere mit zwei oder mehreren Doppelbindungen sind die Ester der Acrylsäure oder Methacrylsäure mit Polyalkoholen mit vorzugsweise 2 bis 6 OH-Gruppen, wie Ethylenglykol, Propylenglykol und deren höher kondensierte Vertreter, wie Diethylenglykol, Triethylenglykol, Dipropylenglykol, Tripropylenglykol etc., Butandiol, Pentandiol, Hexandiol, Neopentylglykol, Bisphenol A, Cyclohexandimethanol, trifunktionelle und höher funktionelle Alkohole, wie Glycerin, Trimethylolpropan, Butantriol, Trimethylolethan, Pentaerythrit, Dimethylolpropan, Dipentaerythrit, Sorbit, Mannit und die entsprechenden alkoxylierten, insbesondere ethoxylierten und propoxylierten Alkohole, sowie Vinylacrylat, Divinylbenzol, Diallylphthalat etc.

Bei den oligomeren bzw. polymeren strahlungshärtbaren Substanzen (strahlungshärtbare Bindemittel) handelt es sich insbesondere um Polyurethane, Polyester, Polyether oder Epoxyharze, welche ethylenisch ungesättigte Doppelbindungen aufweisen. Bevorzugt sind Polyesteracrylate, Polyetheracrylate und insbesondere Polyurethanacrylate (der Ausdruck "Acrylate" soll auch die entsprechenden Methacrylate umfassen), d. h. Ester der Acrylsäure oder Methacrylsäure mit Polyesterolen, Polyetherolen und Polyurethanen mit freien Hydroxygruppen.

Bevorzugte Polyesteracrylate sind aufgebaut aus gesättigten oder ungesättigten Polycarbonsäuren, wie Bernsteinsäure, Adipinsäure, Phthalsäure, Maleinsäure etc., und den oben genannten Polyalkoholen mit vorzugsweise 2 bis 5 OH-Gruppen. Die Polyesteracrylate besitzen vorzugsweise ein zahlenmittleres Molekulargewicht im Bereich von 350 bis 10 000, bestimmt mittels Gelpermeationschromatographie (GPC).

Die Polyetheracrylate sind aufgebaut aus den erwähnten Polyalkoholen und besitzen vorzugsweise ein zahlenmittleres Molekulargewicht im Bereich von 350 bis 10 000, bestimmt mittels GPC.

Urethanacrylate sind erhältlich durch Umsetzung von Polyisocyanaten mit Hydroxyalkyl(meth)acrylaten und gegebenenfalls Kettenverlängerungsmitteln, wie Diole, Polyole, Diamine, Polyamine, Dithiole oder Polythiole. Die Polyurethanacrylate haben vorzugsweise ein zahlenmittleres Molekulargewicht von 1 000 bis 30 000, insbesondere von 1 500 bis 20 000 g/mol (bestimmt durch Gelpermeationschromatographie mit Polystyrol als Standard).

Die Urethanacrylate haben vorzugsweise einen Gehalt von 1 bis 5, besonders bevorzugt 2 bis 4 und ganz besonders bevorzugt 2 bis 3 mol (Meth)acrylgruppen pro 1 000 g Urethanacrylat.

Vorzugsweise sind die Urethanacrylate aufgebaut aus
a) C₁-C₈-Hydroxyalkyl(meth)acrylaten, C₂-C₁₅-Alkandiolen bzw. -polyolen oder C₂-C₈-Alkanolaminen als Kettenverlängerer; sie können auch Polyesterole oder Polyetherole als Aufbaukomponenten enthalten; und
b) aliphatischen Polyisocyanaten, wie Dicyclohexylmethan-4,4'diisocyanat, Hexamethylendiisocyanat, Isophorondiisocyanat, Tetramethylendiisocyanat, Trimethylhexamethylendiisocyanat, Addukten dieser Isocyanate an mehrfunktionelle Alkohole, wie Trimethylolpropan, Di- oder Trimerisationsprodukten der Isocyanate, wie Biurete oder Isocyanurate.

Bevorzugt sind aliphatische Polyisocyanate, wobei der Begriff aliphatisch auch nichtaromatische alicyclische Verbindungen einschließen soll.

Bevorzugte Urethanacrylate sind aliphatische Urethanacrylate, welche aromatische Ringsysteme allenfalls in untergeordneten Mengen von beispielsweise weniger als 5 Gew.-%, bezogen auf die Urethanacrylate, und besonders bevorzugt keine aromatischen Ringsysteme enthalten.

Bevorzugt setzt sich die Gesamtmenge der strahlungshärtbaren Substanzen wie folgt zusammen:
(i) 0,1 bis 10 % eines Phenylglyoxylesters der Formel I und gegebenenfalls 0,1 bis 9,9 % eines weiteren Photoinitiators oder eines Gemisches von Photoinitiatoren, so dass die Gesamtmenge an Photoinitiatoren 10 % nicht überschreitet;
(ii) 20 bis 95 % eines oder mehrerer strahlungshärtbarer Bindemittel, bevorzugt Urethanacrylate, wie oben definiert;
(iii) gegebenenfalls 1 bis 95 % eines oder mehrerer strahlungshärtbarer Reaktivverdünner, die bevorzugt frei von Ethergruppen sind;
(iv) gegebenenfalls 0,1 bis 60 % eines Pigments oder Farbstoffes, gegebenenfalls 0,1 bis 10 % Dispergiermittel;
(v) gegebenenfalls 0,1 bis 10 % weiterer Additive, wie z. B. Verlaufshilfsmittel, Lichtschutzmittel, Mattierungsmittel, Entschäumer, Slippadditive, Amine etc.;
(vi) gegebenenfalls 1 bis 50 % Lösemittel,
jeweils in Gew.-% und bezogen auf das Gesamtgewicht der strahlungshärtbaren Substanz.

Bevorzugt enthalten die strahlungshärtbaren Verbindungen nur geringe oder keine aromatischen Anteile. Der Gehalt an aromatischen Kohlenstoffatomen (d. h. Kohlenstoffatomen, die Bestandteil eines aromatischen Ringsystems sind) liegt vorzugsweise unter 5 Gew.-%, besonders bevorzugt unter 2 Gew.-% und ganz besonders bevorzugt unter 0,5 Gew.-%, bezogen auf die Gesamtmenge der strahlungshärtbaren Verbindungen. Insbesondere beträgt er 0 %.

Die strahlungshärtbaren Substanzen können in den Zusammensetzungen lösungsmittelfrei, in organischen Lösungsmitteln gelöst oder in Wasser dispergiert vorliegen. Lösungsmittel bzw. Wasser liegen dabei im Allgemeinen in einer Menge von 10 bis 70 Gew.-%, bezogen auf das Gesamtgewicht der Zusammensetzung, vor.

Bevorzugt sind lösungsmittelfreie Systeme, d. h. die strahlungshärtbaren Zusammensetzungen liegen in Form eines Pulverlacks vor. Besonders bevorzugt hat der Pulverlack folgende Zusammensetzung:
(i) 0,1 bis 10 % eines Phenylglyoxylesters der Formel I und gegebenenfalls 0,1 bis 9,9 % eines weiteren Photoinitiators oder eines Gemisches von Photoinitiatoren, so dass die Gesamtmenge an Photoinitiatoren 10 % nicht überschreitet;
(ii) 20 bis 95 % eines oder mehrerer strahlungshärtbarer Bindemittel, bevorzugt ein Urethanacrylat, wie oben definiert;
(iii) gegebenenfalls 0,1 bis 60 % eines Pigments oder Farbstoffes, gegebenenfalls 0,1 bis 10 % Dispergiermittel;
(iv) gegebenenfalls 0,1 bis 10 % weiterer Additive, wie z. B. Verlaufshilfsmittel, Weichmacher, Lichtschutzmittel, Mattierungsmittel, Entschäumer, Slippadditive, Amine etc.;
jeweils in Gew.-% und bezogen auf das Gesamtgewicht der strahlungshärtbaren Substanz.

Neben den Photoinitiatoren und strahlungshärtbaren Verbindungen können die strahlungshärtbaren Zusammensetzungen weitere Bestandteile enthalten, insbesondere anorganische und organische Pignente, wie Titandioxid, Zinkoxid, Eisenoxid, Chromoxid oder Azoverbindungen; Bindemittel, wie Polyacrylate; Verlaufsmittel; Adhäsionsverbesserer; Kettenverlängerungsmittel; Füllstoffe, wie Silikate, Carbonate oder Sulfate; Farbstoffe; Netzmittel; Verlaufshilfsmittel und Stabilisatoren. Bei Anwendungen im Außenbereich, d. h. für Beschichtungen, welche dem Tageslicht direkt ausgesetzt sind, enthalten die Massen insbesondere UV-Absorber und Radikalfänger.

UV-Absorber wandeln UV-Strahlung in wärmeenergie um. Brauchbare UV-Absorber sind beispielsweise Hydroxybenzophenone, Benzotriazole, Zimtsäureester und Oxalanilide.

Radikalfänger binden intermediär gebildete Radikale. Brauchbare Radikalfänger sind beispielsweise sterisch gehinderte Amine, welche als HALS (hindered amine light stabilizers) bekannt und z. B. in WO 98/33761 beschrieben sind.

Für Außenanwendungen beträgt der Gehalt an UV-Absorbern und Radikalfängern insgesamt vorzugsweise 0,1 bis 5 Gewichtsteile, besonders bevorzugt 0,5 bis 4 Gewichtsteile, bezogen auf 100 Gewichtsteile der strahlungshärtbaren Verbindungen.

Außerdem können die strahlungshärtbaren Zusammensetzungen auch noch Verbindungen enthalten, die durch andere chemische Reaktionen zur Härtung beitragen. In Betracht kommen beispielsweise Polyisocyanate, welche mit Hydroxyl- oder Amingruppen vernetzen.

Die strahlungshärtbaren Zusammensetzungen eignen sich als Beschichtungsmassen. Es kommen unterschiedliche Substrate in Betracht, beispielsweise Metall, Holz, Papier, Keramik, Glas oder Kunststoff. Es kann sich um Schutzbeschichtungen oder dekorative Beschichtungen handeln. Insbesondere eignen sich die strahlungshärtbaren Zusammensetzungen als Beschichtungsmassen, welche im Außenbereich Anwendung finden, also dem Tageslicht ausgesetzt sind. Derartige Anwendungen sind beispielsweise Außenbeschichtungen von Gebäuden oder Gebäudeteilen, Straßenmarkierungen, Beschichtungen auf Fahrzeugen, wie Personenkraftwagen, Lastkraftwagen, Schienenfahrzeugen und Flugzeugen. Die strahlungshärtbaren Zusammensetzungen eignen sich insbesondere als pigmentfreier Decklack für Kraftfahrzeuge.

Die strahlungshärtbaren Beschichtungsmassen können anhand bekannter Methoden auf die zu beschichtenden Substrate aufgebracht werden. In Betracht kommen insbesondere Auftragsverfahren wie Spritzen, Aufrollen, Aufrakeln etc.

Die Härtung kann durch Bestrahlen mit Licht mit einer Wellenlänge im Bereich von 200 bis 600 nm erfolgen. Vorzugsweise werden jedoch handelsübliche UV-Lampen zur Bestrahlung verwendet.

Pulverlacke werden nach üblichen Pulverauftragsverfahren, beispielsweise elektrostatischem Pulversprühen oder Wirbels intern auf das zu beschichtende Substrat aufgebracht. Im Falle von Holz als Substrat kann beispielsweise vor dem elektrostatischen Versprühen noch ein Leitfähigkeitshilfsmittel aufgebracht werden. Nach dem Aufbringen des Pulverlacks auf das Substrat wird eine Wärmebehandlung bei einer Temperatur im Bereich von 60 bis 100 °C vorgenommen, um den Pulverlack zu einer gleichmäßigen Schicht aufzuschmelzen. Im Anschluss daran erfolgt die Härtung durch Bestrahlung.

Die unter Verwendung der Photoinitiatoren der Formel I hergestellten Beschichtungen und Produkte zeigen eine hohe Beständigkeit gegen Witterungseinflüsse und insbesondere eine geringe Vergilbungsneigung und eine hohe Hydrolysestabilität. Auch die bei der Anwendung von Pulverlacken häufig auftretende Anfangsvergilbung nach der thermischen Behandlung ist verringert. Außerdem hat sich gezeigt, dass sich die erfindungsgemäßen Glyoxalsäureester leicht zu strahlungshärtbaren Zusammensetzungen formulieren lassen. Insbesondere sind sie in den Zusammensetzungen im Allgemeinen klar löslich, während dies bei vergleichbaren Glyoxalsäureestern nicht der Fall ist.

Die nachfolgenden Beispiele erläutern die Erfindung, ohne sie zu beschränken.

### Beispiele

### Beispiel 1: Darstellung von Pentaerythrit-tetraglyoxylat

12,0 g Pentaerythrit, 42,8 g Phenylglyoxylsäure und 0,27 g Schwefelsäure werden in 17,8 g Methylcyclohexan suspendiert und unter Rückfluss erhitzt. Das Wasser der Veresterung wird über ein Trenngefäß entfernt, so dass nach 8 Stunden die Veresterungsreaktion beendet ist. Nach der Destillation des Methylcyclohexans bleibt eine feste Schmelze zurück (Säurezahl 25 mg KOH/g). Gewichtsverlust nach 3 Stunden bei 130 °C: 7,3 %.

### Beispiel 2: Darstellung von 1,4-Butandioldiglycidyletherdiglyoxylat

21,4 g 1,4-Butandioldiglycidylether (Araldit DY026) werden mit 30 g Phenylglyoxylsäure in Gegenwart von 0,5 g Tetrabutylammoniumbromid gemischt. Das Gemisch wird vorsichtig auf 103 °C aufgeheizt und 5 Stunden bei dieser Temperatur gehalten, bis die Säurezahl auf 7,2 mg KOH/g gefallen ist. Das Produkt ist eine hochviskose Flüssigkeit (153,6 Pas). Gewichtsverlust nach 3 Stunden bei 130 °C: 0,7 %.

### Beispiel 3: Darstellung von 1,4-Bis-(phenylglyoxyloxymethyl)-cyclohexan

28,84 g 1,4-Bis-(hydroxymethyl)cyclohexan, 72,23 g Phenylglyoxylsäuremethylester und 0,82 g Dibutoxydibutylzinn werden in einem Reaktionskolben bei 180 °C aufgeschmolzen und über eine Kolonne Methanol abdestilliert. Nachdem nahezu die theoretische Menge Methanol überdestilliert wurde, wird der Rückstand am Rotationsverdampfer einrotiert, in Ethanol digeriert und der entstandene Feststoff abgesaugt. IR- und H-NMR-Spektrum entsprechen der erwarteten Struktur. Schmelzpunkt: 92 °C. Gewichtsverlust nach 3 Stunden bei 130 °C: 0,5 %.

### Beispiel 4

4 Teile 1,4-Cyclohexandimethanoldi(phenylglyoxylat) wurden in 96 Teilen Laromer LR 8987 (eine Mischung aus Hexandioldiacrylat und einem aliphatischen Urethanacrylat) klar gelöst. Über einen Kastenrakel wurde eine Glasplatte mit dieser Formulierung beschichtet, so dass ein Film mit einer Dicke von 50 µm bzw. 100 µm gebildet wird. Anschließend wurde mit 10 m/min unter Luft in einem IST-Bandbelichter gehärtet.

Pendeldämpfung nach DIN 53157 (ISO Norm 1522): 133 sec
Erichsen-Tiefung nach DIN EN ISO 1520: ca. 5 mm
Bleistifthärte: 2H

### Beispiel 5 (Vergleich)

4 Teile 4,4'-Isopropylidendicyclohexanoldi(phenylglyoxylat) [hydriertes Bisphenol-A-di(phenylglyoxylat] konnten in 96 Teilen Laromer LR 8987 nicht gelöst werden. Dadurch war auch eine Strahlungshärtung nicht möglich.

4 Teile 1,4-Cyclohexandioldi(phenylglyoxylat) lösten sich nicht vollständig in 96 Teilen Laromer LR 8987. Die Löslichkeit war jedoch ausreichend, um einen Film wie oben beschrieben herzustellen und zu härten.

Pendeldämpfung nach DIN 53157 (ISO Norm 1522): 141 sec
Erichsen-Tiefung nach DIN EN ISO 1520: ca. 0,6 mm
Bleistifthärte: 2H

### Beispiel 6

a) Löslichkeit in Hexandioldiacrylat (HDDA)

| | 1 % | 2 % | 3 % | 4 % | 5 % | 6 % | 7 % |
|---|---|---|---|---|---|---|---|
| 1,4-Cyclohexandimethanol-di(phenylglyoxylat) | lö | lö | lö | lö | lö | lö | n.l. |
| 4,4'-Isopropylidendicyclohexanoldi(phenylglyoxylat) | lö | n.l. | | | | | |
| 1,4-Cyclohexandiol-di(phenylglyoxylat) | lö | lö | n.l. | | | | |
| (n.l. = nicht löslich, lö = löslich) | | | | | | | |

b) weiterhin wurde die Löslichkeit von je 2 % 1,4-Cyclohexandimethanoldi(phenylglyoxylat) und 4,4'-Isopropylidendicyclohexanoldi(phenylglyoxylat) in einem aliphatischen Urethanacrylat geprüft. Das 1,4-Cyclohexandimethanoldi(phenylglyoxylat) war löslich, während das 4,4'-Isopropylidendicyclohexanoldi(phenylglyoxylat) sich nicht oder nicht vollständig löste.

### Beispiel 7

In einer Rührapparatur werden 204,2 T (T = Teile) Isophorondiisocyanat und 0,3 T Dibutylzinndilaurat vorgelegt und bei 60 °C 91,64 T Hydroxyethylacrylat, 36,00 T Butandiol und 11,80 T Trimethylolpropan zugegeben.

Es erfolgt exothermer Temperaturanstieg bis ca. 110 °C. Anschließend wird auf 135 °C erwärmt und diese Temperatur 10 Minuten gehalten, dann wird auf ca. 100 °C gekühlt. 11,00 T Cyclohexandimethanoldiphenylglyoxylat (Photoinitiator) werden eingerührt, die Schmelze wird auf Aluminiumfolie gegossen, nach dem Erstarren gemahlen und auf 40 µm abgesiebt. Das Pulver wird in einer Pulverkabine elektrostatisch auf Blechtafeln gesprüht, unter einer IR-Lampe rasch auf 130 °C erwärmt und mit 40 m/min UV-belichtet. Nach dem Abkühlen resultieren harte Lackfilme mit guter Lösemittelbeständigkeit (> 50 Hübe/MEK).

## Patentansprüche

1. Verwendung von Phenylglyoxalsäureestern der Formel I 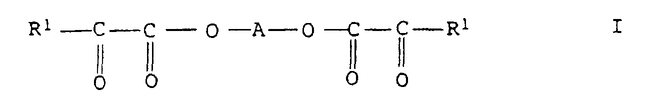 worin die beiden Reste R¹ unabhängig voneinander für einen Rest der Formel 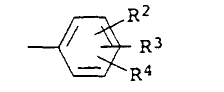 stehen,
R², R³ und R⁴ unabhängig voneinander für H, C₁-C₆-Alkyl, das gegebenenfalls durch OH, OC₁-C₆-Alkyl oder OCOC₁-C₆-Alkyl substituiert ist, oder für OH oder OC₁-C₆-Alkyl stehen;
A für C₂-C₆-Alkylen oder einen Rest der Formeln 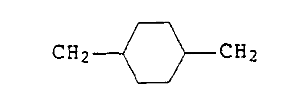 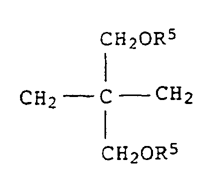 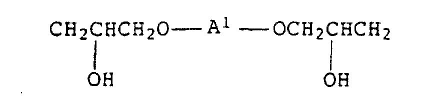 steht,
die Reste R⁵ unabhängig voneinander für H oder COCOR¹ stehen und
A¹ für C₂-C₆-Alkylen oder 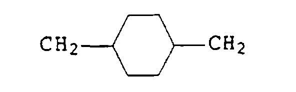 steht,
als Photoinitiatoren in Pulverlacken für Außenanwendungen.

2. Verwendung nach Anspruch 1 von Phenylglyoxalsäureestern der Formel I, worin A für 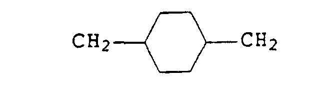 oder 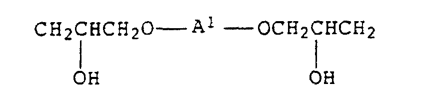 steht, und
A¹ für 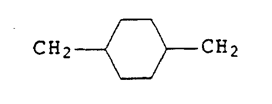 steht.

3. Verwendung nach Anspruch 1 oder 2 zur Außenbeschichtung von Gebäuden oder Gebäudeteilen und Beschichtungen auf Fahrzeugen oder Flugzeugen.

4. Phenylglyoxalsäureester der Formel I 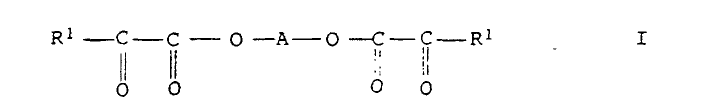 worin R¹ für 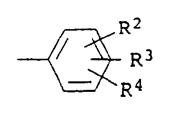 steht;
A für 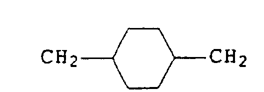 oder 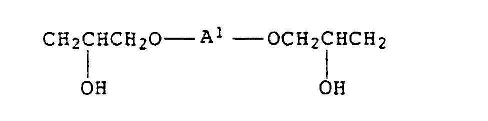 steht; und
A¹ für 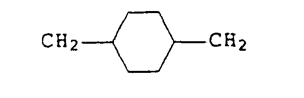 steht.

5. Phenylglyoxalsäureester nach Anspruch 4 der Formel I, worin R², R³ und R⁴ unabhängig voneinander für H oder C₁-C₆-Alkyl stehen.

6. Strahlungshärtbare Zusammensetzung, enthaltend als Photoinitiator wenigstens einen Phenylglyoxalsäureester nach Anspruch 4 oder 5.

7. Zusammensetzung nach Anspruch 6 in Form eines Pulverlackes.

8. Verfahren zur Beschichtung von Substraten, **dadurch gekennzeichnet, dass** man auf die Substrate eine Zusammensetzung nach Anspruch 6 oder 7 aufträgt, bei Verwendung eines Pulverlackes nach Anspruch 7 eine wärmebehandlung vornimmt, um den Pulverlack aufzuschmelzen, und das beschichtete Substrat mit Licht einer Wellenlänge im Bereich von 200 bis 600 nm bestrahlt.

9. Beschichtetes Substrat, das zumindest teilweise mit einer Zusammensetzung nach Anspruch 6 oder 7 beschichtet ist.

10. Beschichtetes Substrat, erhältlich nach Anspruch 8.

## Claims

1. The use of phenylglyoxalic esters of the formula I 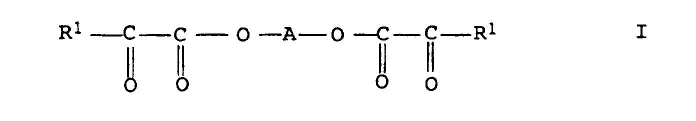 in which the two radicals R¹ independently of one another are a radical of the formula 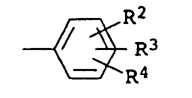 R², R³ and R⁴ independently of one another are H, C₁-C₆-alkyl, which is unsubstituted or substituted by OH, OC₁-C₆-alkyl or OCOC₁-C₆-alkyl, or are OH or OC₁-C₆-alkyl;
A is C₂-C₆-alkylene or a radical of the formulae 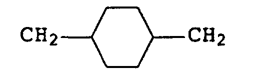 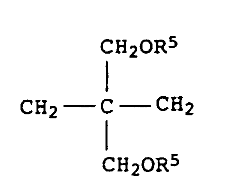 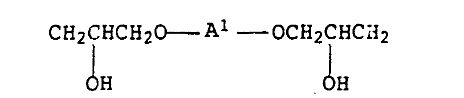 the radicals R⁵ independently of one another are H or COCOR¹, and
A¹ is C₂-C₆-alkylene or 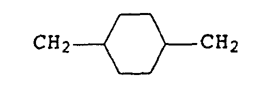 as photoinitiators in powder coating materials for exterior applications.

2. The use as claimed in claim 1 of phenylglyoxalic esters of the formula I in which A is 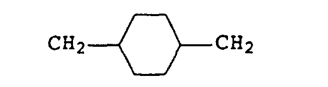 or 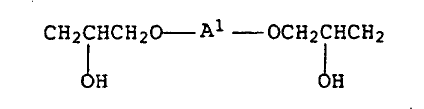 and
A¹ is

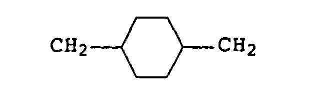

3. The use as claimed in claim 1 or 2 for the exterior coating of buildings or parts of buildings and coatings on vehicles or aircraft.

4. A phenylglyoxalic ester of the formula I 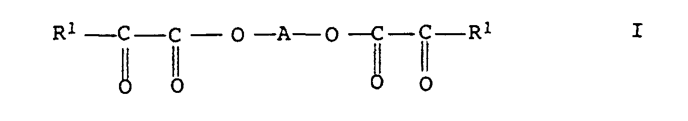 in which R¹ is 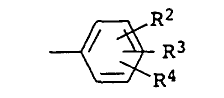 A is 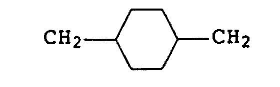 or 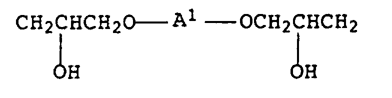 and
A¹ is

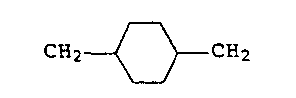

5. A phenylglyoxalic ester as claimed in claim 4 of the formula I in which R², R³ and R⁴ independently of one another are H or C₁-C₆-alkyl.

6. A radiation-curable composition comprising as photoinitiator at least one phenylglyoxalic ester as claimed in claim 4 or 5.

7. A composition as claimed in claim 6 in the form of a powder coating material.

8. A method of coating a substrate, which comprises applying to said substrate a composition as claimed in claim 6 or 7, performing a heat treatment if using a powder coating material as claimed in claim 7, in order to melt the powder coating material, and irradiating the coated substrate with light having a wavelength in the range from 200 to 600 nm.

9. A coated substrate coated at least partly with a composition as claimed in claim 6 or 7.

10. A coated substrate obtainable as claimed in claim 8.

## Revendications

1. Utilisation d'esters d'acide phénylglyoxalique de la formule I : 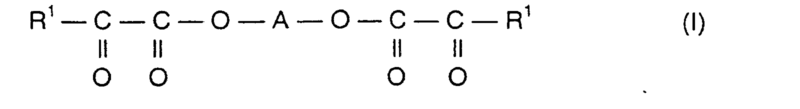 dans laquelle les deux radicaux R¹ représentent, indépendamment l'un de l'autre, un radical de la formule : 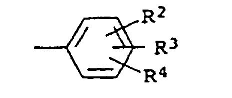 où R², R³ et R⁴ représentent, indépendamment l'un de l'autre, H, de l'alkyle en C₁-C₆, qui est éventuellement substitué par OH, du O-alkyle en C₁-C₆ ou du OCO-alkyle en C₁-C₆, ou OH ou du O-alkyle en C₁-C₆,
A représente un alkylène en C₂-C₆ ou un radical des formules : 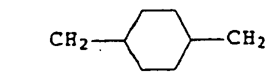 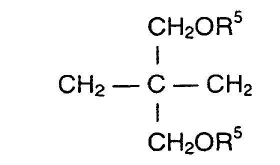 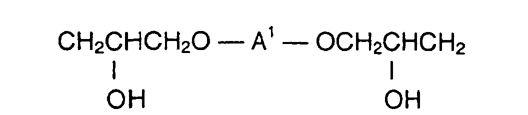 où les radicaux R⁵ représentent, indépendamment l'un de l'autre, H ou COCOR¹, et
A¹ représente de l'alkylène en C₂-C₆ ou 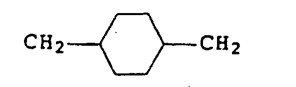 comme photo-initiateurs dans des laques en poudre destinées à des applications extérieures.

2. Utilisation suivant la revendication 1 d'esters d'acide phénylglyoxalique de la formule I, dans laquelle A représente 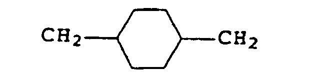 ou 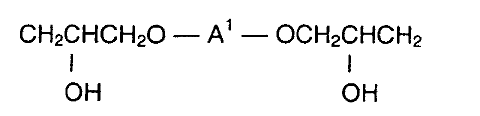 et
A¹ représente

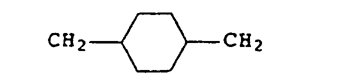

3. Utilisation suivant l'une des revendications 1 et 2, pour le recouvrement extérieur de bâtiments ou de parties de bâtiments et pour des revêtements sur des véhicules ou des avions.

4. Esters d'acide phénylglyoxalique de la formule 1 : 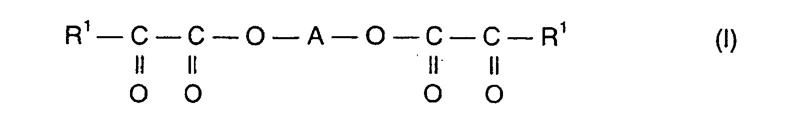 dans laquelle R¹ représente 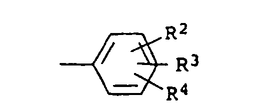 A représente 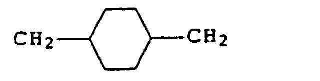 ou 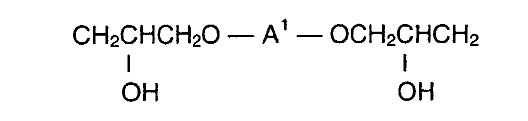 et
A¹ représente

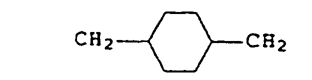

5. Esters d'acide phénylglyoxalique suivant la revendication 4 répondant à la formule 1, dans lesquels R², R³ et R⁴ représentent, indépendamment l'un de l'autre, H ou de l'alkyle en C₁-C₆.

6. Composition durcissable au rayonnement, contenant comme photo-initiateur au moins un ester d'acide phénylglyoxalique suivant l'une des revendications 4 et 5.

7. Composition suivant la revendication 6, sous la forme d'une laque en poudre.

8. Procédé de revêtement de substrats, **caractérisé en ce qu'**on applique sur les substrats une composition suivant l'une des revendications 6 et 7, lors de l'utilisation d'une laque en poudre suivant la revendication 7, on effectue un traitement thermique pour faire fondre la laque en poudre, et on irradie le substrat revêtu par une lumière d'une longueur d'onde de l'ordre de 200 à 600 nm.

9. Substrat revêtu, qui est enduit au moins partiellement d'une composition suivant l'une des revendications 6 et 7.

10. Substrat revêtu, que l'on peut obtenir selon la revendication 8.
